# FASCICULE DE BREVET EUROPEEN

(11) **EP 0 574 301 B1**
(45) Date de publication et mention de la délivrance du brevet: **14.08.1996**
(21) Numéro de dépôt: 93401457.2
(22) Date de dépôt: 08.06.1993
(51) Int. Cl.: A61K 9/20, A61K 9/00

(54) **Pastille permettant l'administration des comprimés aux animaux domestiques**
Pastille zur Verabreichung von Tabletten an Haustiere
Pastille for delivering tablets to domestic animals

(30) Priorité: 09.06.1992 FR 9206908
(43) Date de publication de la demande: 15.12.1993
(73) Titulaire: SOGEVAL S.A., FR-53031 Laval, Mayenne (FR)
(72) Inventeur: Daoudal, José, Laval (Mayenne) (FR)
(74) Mandataire: Cabinet HERRBURGER

(56) Documents cités:
- EP-A- 0 320 320
- FR-A- 2 073 278

## Description

La présente invention concerne une pastille permettant de faciliter l'absorption par des animaux domestiques, en particulier des chiens ou des chats, de comprimés notamment médicamenteux à odeur et goût répulsifs pour ces animaux.

Dans ce qui suit, le terme pastille devra être compris dans un sens large et n'est pas limité au seul domaine des médicaments.

Le nombre des animaux domestiques tels que chiens et chats est en augmentation constante. Parallèlement à cette augmentation, on assiste à une évolution des soins apportés à ces animaux.

Il faut constater, qu'à ce jour, le suivi des traitements administrés n'est pas toujours bien assuré en raison de la difficulté à faire absorber les médications à l'animal.

Il est, en effet, bien connu que de nombreuses substances médicamenteuses exercent un effet répulsif pour l'odorat des chiens ou des chats qui refusent de les absorber, rendant ainsi très difficile, voire impossible, le respect d'un traitement.

Pour résoudre ce problème, on a déjà proposé, selon le document EP-0 320 320, un comprimé constitué par au moins un noyau contenant un ou plusieurs principes actifs exerçant un effet répulsif pour l'odorat de l'animal, totalement englobé dans une matrice appétente.

Ce comprimé s'est révélé satisfaisant en ce qu'il est susceptible d'être absorbé spontanément par l'animal, mais, présente parallèlement, l'inconvénient d'être particulièrement onéreux, compte tenu de la nécessité de prévoir un processus d'enrobage spécifique pour chaque type de comprimé, entraînant une augmentation notable du coût des traitements vétérinaires.

De plus, pour que tous les propriétaires de chiens et de chats puissent profiter des avantages de ce comprimé, il faudrait généraliser sa fabrication, et donc, faire en sorte que tous les laboratoires de fabrication de comprimés notamment médicamenteux destinés aux chiens ou aux chats, prévoient un procédé d'enrobage, ce qui est difficilement envisageable ; en outre, il existe, actuellement sur le marché, un « stock » de comprimés qui ne peuvent pas être enrobés.

Il est à noter que l'on a déjà proposé, conformément au document FR-A-2 073 278, une pastille composite constituée par l'association d'au moins deux comprimés, tous actifs - à savoir un comprimé central et au moins un comprimé périphérique - qui est obtenue par la mise en oeuvre d'un processus d'enrobage particulier ; cette pastille ne comporte pas de logement susceptible de recevoir un comprimé actif complémentaire.

La présente invention a pour objet de remédier à ces inconvénients en proposant une pastille permettant de faciliter l'absorption de comprimés notamment médicamenteux par des animaux domestiques, notamment des chiens ou des chats, grâce à laquelle l'opération d'enrobage puisse, en quelque sorte, être effectuée par le propriétaire de l'animal lui-même, au moment de l'administration du principe actif.

Cette pastille est caractérisée en ce qu'elle est constituée par une matrice en un matériau appétent comportant deux faces principales dont l'une est munie d'un logement ouvert vers l'extérieur ayant une forme et des dimensions correspondant à celles du comprimé à administrer de façon à permettre, préalablement à l'absorption, l'introduction à force et le maintien de ce comprimé sur cette face sur laquelle il demeure visible, tandis que l'autre face principale essentiellement lisse est destinée à être présentée à l'animal.

On s'est en effet aperçu de manière surprenante que la pastille conforme à l'invention est absorbée spontanément par les chiens et les chats, et ce, bien que le comprimé actif demeure visible sur la face arrière de la pastille.

On peut, bien entendu, prévoir de maintenir le comprimé dans le logement, de façon quelconque, notamment grâce à une substance adhérente.

Selon une caractéristique préférentielle de l'invention, le logement a une forme tronconique de façon à permettre l'introduction et le maintien dans la matrice appétente de comprimés de dimensions différentes.

Il est clair que la pastille conforme à l'invention est de nature à diminuer notablement le coût des traitements médicamenteux des chiens et des chats. Chaque propriétaire d'animal peut, en effet, acquérir un lot de pastilles qu'il « équipera » de comprimés au fur et à mesure de ses besoins.

De façon connue en elle-même on s'est rendu compte, en travaillant les facteurs d'appétence pour chiens et chats, que la poudre de foie est particulièrement prisée par ces animaux et qu'un mélange poudre de foie/levure de bière est de nature à faciliter, dans une large mesure, l'absorption des médicaments par les chiens et les chats, permettant par suite, de leur administrer facilement des traitements préventifs ou curatifs.

Pour cette raison, la matrice appétente conforme à l'invention peut, avantageusement, renfermer un mélange de poudre de foie et de levure de bière. Plus précisément, on peut utiliser, pour la fabrication de cette matrice, les matériaux cités dans le document EP-0 320 320 susmentionné.

La forme de la pastille conforme à l'invention n'est, bien entendu, pas limitative de l'invention : on opte, cependant généralement, pour la forme d'un disque et en particulier d'un disque dont les deux faces principales présentent une légère convexité, cette forme qui correspond à la forme de la grande majorité des comprimés notamment médicamenteux, s'étant révélée particulièrement avantageuse pour des raisons de facilité de fabrication.

Les caractéristiques de la pastille qui fait l'objet de l'invention seront décrites plus en détail en se référant aux dessins annexés dans lesquels :
- la figure 1 est une vue en perspective de la pastille avant mise en place du comprimé,
- les figures 2 et 3 sont des vues en coupe par un plan diamétral d'une pastille « équipée » d'un comprimé.

Il est à noter que l'échelle des figures est largement agrandie, et que pour correspondre aux dimensions des comprimés usuels du commerce, les pastilles ont, de préférence, un diamètre de l'ordre de 20 mm.

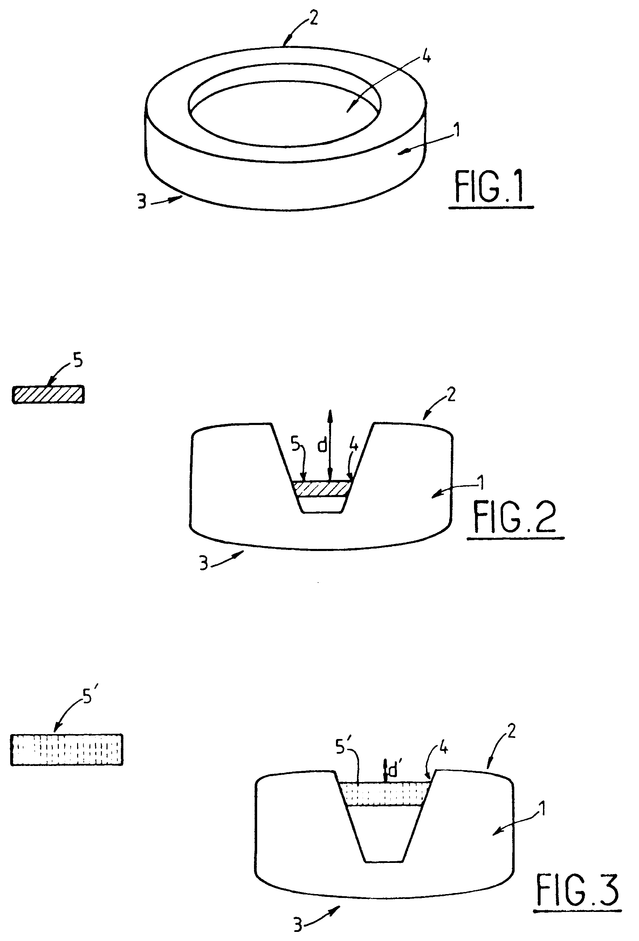

Selon les figures 1 à 3, la pastille 1 conforme à l'invention est constituée par une matrice en forme de disque réalisée en un matériau renfermant une mélange de poudre de foie et de levure de bière qui s'est révélé comme étant particulièrement appétent pour des animaux domestiques tels que des chiens et des chats.

Cette matrice 1 comporte deux faces principales 2 et 3 qui présentent une légère convexité.

Conformément à l'exemple non limitatif représenté sur les figures 2 et 3, la face principale 2 est munie d'un logement 4 de forme tronconique destiné à recevoir un comprimé notamment médicamenteux 5, 5' (figures 2 et 3) introduit à force par le propriétaire de l'animal au moment de l'administration du médicament.

Lors de l'utilisation, la pastille est présentée à l'animal, face 3 tournée vers lui et peut, ainsi, être absorbée sans problème.

Grâce au choix d'une forme tronconique, le logement 4 peut s'adapter à des comprimés de différentes dimensions : le comprimé 5' introduit dans le logement 4, selon la figure 3, est nettement plus gros que le comprimé représenté sur la figure 2 et peut néanmoins être parfaitement maintenu.

La profondeur d, d' de pénétration du comprimé 5, 5' dans le logement 4 dépend bien entendu de ses dimensions et est moins importante dans le cas d'un gros comprimé.

## Revendications

1. Pastille permettant de faciliter l'absorption par des animaux domestiques, en particulier des chiens ou des chats, de comprimés notamment médicamenteux à odeur et goût répulsifs pour ces animaux, caractérisée en ce qu'elle est constituée par une matrice (1) en un matériau appétent comportant deux faces principales (2, 3) dont l'une (2) est munie d'un logement (4) ouvert vers l'extérieur ayant une forme et des dimensions correspondant à celles du comprimé (5) à administrer de façon à permettre, préalablement à l'absorption, l'introduction à force et le maintien de ce comprimé (5) sur cette face (2) sur laquelle il demeure visible, tandis que l'autre face principale (3) essentiellement lisse est destinée à être présentée à l'animal.

2. Pastille selon la revendication 1, caractérisée en ce que le logement (4) a une forme tronconique de façon à permettre l'introduction et le maintien dans la matrice (1) appétente de comprimés de dimensions différentes.

3. Pastille selon l'une quelconque des revendications 1 et 2, caractérisée en ce que la matrice (1) appétente renferme un mélange de poudre de foie et de levure de bière.

4. Pastille selon l'une quelconque des revendications 1 à 3, caractérisée en ce qu'elle a essentiellement la forme d'un disque.

5. Pastille selon l'une quelconque des revendications 1 à 4, caractérisée en ce que les deux faces principales (2, 3) présente une légère convexité.

## Claims

1. A pastille for facilitating the absorption by domestic animals, and particularly by dogs and cats, of notably medicinal tablets with a smell and taste which these animals find repulsive, characterised in that it is made up of a matrix (1) of a palatable material comprised of two main faces (2, 3), one of which (2) has a recess (4) open to the outside with a form and dimensions corresponding to those of the tablet (5) to be administered so that, prior to absorption, this tablet (5) may be pressed into and held on this face (2) on which it remains visible, while the other, essentially smooth, main face (3) is designed to be presented to the animal.

2. A pastille as claimed in Claim 1, characterised in that the recess (4) is of a tapered form, so that tablets of various dimensions may be introduced into and held in the matrix (1).

3. A pastille as claimed in either of Claims 1 or 2, characterised in that the palatable matrix (1) contains a mixture of liver powder and brewer's yeast.

4. A pastille as claimed in any of Claims 1 to 3, characterised in that it is essentially in the form of a disk.

5. A pastille as claimed in any of Claims 1 to 4, characterised in that both main faces (2, 3) are slightly convex in form.

## Patentansprüche

1. Pastille zur Erleichterung der Verabreichung von Tabletten, nämlich Medikamenten mit für Tiere abstoßendem Geruch und Geschmack, an Haustiere, insbesondere an Hunde und Katzen,
**dadurch gekennzeichnet**,
daß die Pastille aus einer Matrize (1) aus instinktmäßig begehrtem Stoff gebildet ist, die zwei Hauptseiten (2, 3) aufweist, von denen eine Hauptseite (2) mit einer nach außen offenen Aufnahme (4) ausgestattet ist, die eine Form und Abmessungen entsprechend der zu verabreichenden Tablette (5) aufweist, so daß vor der Verabreichung das Einsetzen mit Kraft und das Halten der Tablette (5) auf der Seite (2), auf der sie sichtbar bleibt, möglich ist, wohingegen die andere, im wesentlichen glatte Hauptseite (3) dazu bestimmt ist, dem Tier angeboten zu werden.

2. Pastille nach Anspruch 1,
**dadurch gekennzeichnet**,
daß die Aufnahme (4) eine kegelstumpfförmige Form aufweist, so daß das Einsetzen und das Halten von Tabletten mit verschiedenen Abmessungen in der instinktmäßig begehrten Matrize (1) ermöglicht wird.

3. Pastille nach einem der Ansprüche 1 und 2,
**dadurch gekennzeichnet**,
daß die instinktmäßig begehrte Matrize (1) eine Mischung aus Leber- und Bierhefepulver einschließt.

4. Pastille nach einem der Ansprüche 1 bis 3,
**dadurch gekennzeichnet**,
daß die Pastille im wesentlichen die Form einer Scheibe aufweist.

5. Pastille nach einem der Ansprüche 1 bis 4,
**dadurch gekennzeichnet**,
daß die zwei Hauptseiten (2, 3) eine leichte Wölbung aufweisen.
